# EUROPÄISCHE PATENTSCHRIFT

(11) **EP 3 065 802 B1**
(45) Veröffentlichungstag und Bekanntmachung des Hinweises auf die Patenterteilung: **28.06.2017**
(21) Anmeldenummer: 14796705.3
(22) Anmeldetag: 03.11.2014
(51) Int. Cl.: A61M 16/04, A61F 2/20, A61M 16/10

(54) **SPRECHVENTIL FÜR LARYNGEKTOMIERTE ODER TRACHEOTOMIERTE PATIENTEN**
SPEECH VALVE FOR PERSONS HAVING UNDERGONE A LARYNGECTOMY OR TRACHEOTOMY
VALVE PHONIQUE POUR DES PATIENTS LARYNGECTOMISÉS OU TRACHÉOTOMISÉS

(30) Priorität: 04.11.2013 DE 102013018423
(43) Veröffentlichungstag der Anmeldung: 14.09.2016
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2014/002934
(87) Internationale Veröffentlichungsnummer: WO 2015/062741

(56) Entgegenhaltungen:
- EP-A1- 2 236 165
- WO-A1-2011/144237
- WO-A2-2012/048681
- DE-T2- 69 920 440
- DE-U1-202012 001 825
- DE-U1-202013 001 950
- DE-U1-202013 008 092
- US-A- 5 738 095

## Beschreibung

Die vorliegende Erfindung betrifft ein Sprechventil für Laryngektomierte oder Tracheotomierte mit mindestens einem Gehäuse mit einer proximalen, das heißt körpernahen Öffnung, und mindestens einem im Gehäuse zumindest teilweise aufgenommenen Filter.

Sprechventile der eingangs genannten Art sind vielfältig bekannt, und verbinden zwei wesentliche Eigenschaften in einer einzigen Vorrichtung, nämlich einerseits dienen sie der Befeuchtung der Atemluft, andererseits ermöglichen sie bei Überführung aus einer Offenstellung in einer Schließstellung ein Sprechen eines Laryngektomierten oder Tracheotomierten.

Bei operativen Eingriffen im oberen Atemtrakt kann das Anlegen einer künstlichen Atemöffnung (Tracheostoma) in der Luftröhre notwendig sein, damit unter Umgehung von Mundraum und Kehlkopf Luft direkt in die Lunge eingeatmet werden kann. Bei Laryngektomierten oder Tracheotomierten werden üblicherweise Filtersysteme eingesetzt, welche aus einem Pflaster mit einem eingesetzten Filter, aus einer üblicherweise selbstklebenden Basisplatte oder einer Trachealkanüle bestehen - in aller Regel aus Kunststoff gebildet -, in welche Filter unterschiedlichster Art einsetzbar sind. Zu den Filtersystemen, welche für laryngotracheale Hilfsmittel wie Trachealkanülen und Pflaster- oder Basisplatte eingesetzt werden, zählen die sogenannten Feuchte- und Wärmeaustauscher, auch künstliche Nasen genannt. Diese dienen dazu, die bei tracheotomierten, aber auch bei laryngektomierten Patienten fehlenden Regulationsmechanismen zur Erwärmung und Befeuchtung der Atemluft nachzubilden und ein Inkontaktbringen der Luftröhre mit trockner, kalter und nichtgefilterter Luft zu vermeiden. Denn durch die hierdurch hervorgerufene Reizung tritt eine erhöhte Schleimproduktion mit der nachfolgenden Gefahr der Verborkung auf. Durch Feuchte- und Wärmeaustauscher wird nun die eingeatmete Luft angefeuchtet, erwärmt und gleichzeitig gefiltert. Hierdurch wird die vorstehend erwähnte Borkenbildung weitgehend vermieden. Dabei hilft das regelmäßige Tragen der künstlichen Nase insbesondere bei starker Sekretabsonderung, da durch das Anfeuchten der Schleimhäute in der Luftröhre die Sekretproduktion vermindert wird. Feuchte- und Wärmeaustauscher können darüber hinaus auch mit einer Sprechfunktion ausgestattet sein, und werden dann, so auch im Sinne der vorliegenden Erfindung, als Sprechventil angesprochen, wobei jedoch andere Bezeichnungen wie Beispiel Stimmventil ebenfalls gängig sind.

Die DE 699 20 440 T2 offenbart eine gattungsgemäße, dort als Stimmventil mit Filter bezeichnete Vorrichtung, wobei das Stimmventil der Verbindung mit einem Tracheostoma dient, und einen regenerativen Filter aufweist zum Feuchtigkeits- und Wärmeaustausch beim Atmen durch das Stimmventil, sowie ein Gehäuse, das den Filter aufnimmt, und eine erste Öffnung an einer Seite von dem Filter zur Verbindung mit dem Tracheostoma sowie zumindest eine zweite Öffnung einer gegenüberliegenden Seite von dem Filter, die mit der Umgebung verbunden ist, und ein manuell betätigbares Ventilelement zum Blockieren eines Luftdurchganges durch den Filter, wobei eine Hülse in das Innere von dem Gehäuse ragt und einen Ventilsitz bildet, welcher die erste Öffnung definiert, um mit dem Ventilelement durch eine manuelle Betätigung von diesem abdichtend zum Eingriff zu kommen.

Sämtliche der in der DE 699 20 440 T2 offenbarten Vorrichtungen bedingen, dass bei einer Schließung der proximalen Öffnung der Filter zumindest teilweise komprimiert wird. Aufgrund der Elastizität des Filtermateriales erfolgt dann eine Überführung aus der Schließstellung in eine Offenstellung des dort offenbarten Stimmventiles. Nachteilig hieran ist insbesondere, dass einerseits eine Ermüdung des Filtermateriales durch die Komprimierung und Dekomprimierung während der Überführung in die Schließstellung und Offenstellung des Stimmventils erfolgt, andererseits kann auch die elastische Eigenschaft des Filtermateriales im Gebrauch nachlassen, so dass ein sicheres Öffnen und Verschließen nicht mehr gegeben ist.

WO 2011/144237 A1 beschreibt eine Filtervorrichtung zur Verwendung bei einem Tracheostoma. Die Filtervorrichtung weist ein Gehäuse mit einer ersten und einer zweiten Öffnung auf. Des Weiteren ist die erste Öffnung mittels eines Verschlusselementes verschließbar. Weiterhin weist die Filtervorrichtung ein Betätigungselement auf, welches in Wirkverbindung mit einem Federelement zur automatischen Bewegung des Verschlusselementes in dessen Öffnungsposition ist.

DE 20 2013 008 092 U1 beschreibt eine Halterung für Sprechventile und/oder Wärme-Feuchtigkeitstauscher. Ein Filter mit einer oberen und unteren Verhautung ohne ein Gehäuse schließt durch Fingerdruck die Luftöffnung mittels Dichtplatte oder öffnet ohne Fingerdruck, um einen Sprechvorgang oder Atemvorgang zu ermöglichen.

EP 2 236 165 A1 beschreibt ein Tracheostomaventil, welches eine Ventilanordnung mit einem zumindest teilweise flexiblen Wandteil mit einer umlaufenden Grenzkante aufweist. Diese Grenzkante formt ein Gelenk für das Wandteil.

Die vorliegende Erfindung hat die Aufgabe, ein gattungsgemäßes Sprechventil zur Verfügung zu stellen, bei welchem die aus dem Stand der Technik bekannten Nachteile vermieden werden, und welches insbesondere langlebig ausgebildet ist und nur der Anwendung geringer Kräfte bei einer Überführung aus einer Offen- in eine Schließstellung bei gleichzeitig sicherer Funktion bedarf.

Diese Aufgabe wird gelöst durch ein erfindungsgemäßes Sprechventil der eingangs genannten Art, wobei dieses weiterhin umfasst ein kolbenartig ausgebildetes Ventilelement, wobei der Filter das Ventilelement zumindest teilweise umgibt, wobei das Ventilelement auf einen an der proximalen Öffnung angeordneten Ventilsitz zur Erzeugung einer Schließstellung bewegbar ist und federnd in eine Offenstellung des Sprechventiles rückführbar ist, wobei das Gehäuse ein Deckelteil umfasst, welches eine Öffnung für das Ventilelement aufweist, wobei das Ventilelement in Offenstellung des Sprechventiles dicht an der Öffnung anliegt, und wobei das Ventilelement ein unteres, der proximalen Öffnung zugewandtes Ende aufweist, welches in Offenstellung dem Ventilsitz zugeordnet ist und mit dem Ventilsitz einen Spalt, insbesondere Ringspalt, der groß genug ist, um die Atemfunktion zu erfüllen, bildet, durch den ein Luftstrom führbar ist. Das Gehäuse des erfindungsgemäßen Sprechventils umfasst ein Deckelteil, und vorzugsweise auch ein Bodenteil. Bevorzugt weist das Gehäuse zumindest eine umlaufende Seitenwand auf. Diese kann Öffnungen aufweisen. Die mindestens eine Seitenwand umfasst den Filter zumindest teilweise. Der Filter ist zumindest teilweise im Gehäuse aufgenommen. Das kolbenartig ausgebildete Ventilelement ist einer Öffnung in der Oberseite des Deckelteils zugeordnet, und kann dabei auch durch diese Öffnung herausragen. Es ist überwiegend im Gehäuse aufgenommen. Bei den vorgenannten Ausführungsformen ist eine Betätigung durch den Benutzer mittels eines Fingers ermöglicht, indem entweder bei einer Zuordnung zu einer Öffnung unmittelbar mit dem Finger das kolbenartige Ventilelement in Kontakt bringbar und daher auch in Richtung auf die proximale Öffnung zubewegbar ist, oder aber der Finger des Benutzers nur mittelbar mit dem kolbenartigen Ventilelement in Kontakt kommt, wenn beispielsweise zumindest ein Teilbereich einer Oberseite des Deckelteiles aus einem elastischen Kunststoffmaterial gebildet ist, welches mit der Oberseite des Ventilelementes in Kontakt steht oder dieser nahe angeordnet ist, so dass durch eine Bewegung dieses elastischen Teiles der Oberseite des Deckelteiles eine Bewegbarkeit des Ventilelementes auf die proximale Öffnung zu ermöglicht ist. Besonders bevorzugt weist das kolbenartig ausgebildete Ventilelement ein Bedienelement auf, welches auch der proximalen Öffnung zugewandt am Ventilelement angeordnet ist, und dem Benutzer ein korrektes Erspüren und damit auch Bedienen des Ventilelementes ermöglicht. Beispielsweise kann dabei das Bedienelement im Querschnitt gesehen halbrund, in der Aufsicht gesehen kreisrund ausgebildet sein, und weiter bevorzugt bei Vorsehung einer insbesondere zentralen Öffnung in der Oberseite des Deckelteils durch diese zumindest teilweise herausragen, um eine Bedienung des erfindungsgemäßen Sprechventiles für den Benutzer weiter zu erleichtern.

Die federnde Rückführung des Ventilelementes in eine Offenstellung des Sprechventiles erfolgt vorteilhafterweise durch Vorsehung mindestens eines Federelementes. Dies kann als Druckfeder- und/oder Zugfederelement ausgebildet sein. Das mindestens eine Federelement kann aber auch, obwohl nicht bevorzugt, zum Beispiel auch aus einem elastischen Material gebildet sein, beispielsweise aus einem geeigneten Kunststoffmaterial, das bevorzugt geschäumt ausgebildet ist. Dieses ist eine Alternative zu einem üblichen Druckfeder- und/oder Zugelement. Dabei kann das elastische Material ebenfalls als Filtermaterial ausgebildet sein. Es kann dabei sowohl als üblicher Fein- aber auch als Grobfilter ausgebildet sein. Durch Verwendung eines insbesondere geschäumten Filtermaterials kann eine Doppelfilterung erfolgen und dadurch der Trage-Komfort für den Benutzer verbessert werden. Der eigentliche Filter, der das Ventilelement zumindest teilweise umgibt, ist vorzugsweise aus einem geschäumten Material, insbesondere aus Kunststoff, gebildet, kann aber auch aus Vliesmaterialien, Papier oder ähnlichem gebildet sein. Er kann als Fein- und/oder Grobfilter ausgebildet sein, und insbesondere als Grobfilter, wenn als Federelement ebenfalls ein filterartiges Material eingesetzt wird, welches dann bevorzugt als Feinfilter ausgebildet ist. Ein Grobfilter hat im Vergleich zu einem Feinfilter insbesondere größere Poren/Öffnungen, so dass der Luftdurchgang mit geringerem Widerstand erfolgt als bei einem Feinfilter. Das Material des Filters und/oder des aus einem filterartigen Materal gebildeten Federelementes kann dabei beschichtet und/oder getränkt sein, beispielsweise mit antistatisch und/oder antibakteriell wirkenden Zusammensetzungen.

Das Ventilelement ist bevorzugt über mindestens ein Federelement im Gehäuse gelagert. Das mindestens eine Federelement kann in jeder Art und Weise ausgebildet sein. Es kann insbesondere als elastisches geschäumtes Material, wie oben beschrieben, wenn auch nicht bevorzugt, insbesondere aber als Schraubenfeder, Schraubenzugfeder, Kegelfeder, Spiralfeder, Tellerfeder, Ringfeder oder Luftfeder ausgebildet sein. Je nach Ausgestaltung des mindestens einen Federelementes, insbesondere als Druckfederelement, sind entsprechend Lagerungen vorzusehen, die dem Federelement einen sicheren Halt oder Anordnung zur Verfügung stellen. Die federnde Lagerung kann dabei vorzugsweise an einem proximalen ersten Ende des erfindungsgemäßen Sprechventiles, an welchem auch die proximale Öffnung angeordnet ist, oder an einem diesen gegenüberliegenden distalen, das heißt körperfernen zweiten Ende des erfindungsgemäßen Sprechventiles, an welchem auch der Deckelteil des Gehäuses vorhanden ist, angeordnet sein. Vorteilhafterweise sind Lagermittel am kolbenartig ausgebildeten Ventilelement vorgesehen, welchen Gegenlager im Bereich des proximalen ersten Endes und/oder des distalen zweiten Endes des erfindungsgemäßen Sprechventiles zugeordnet sind. Das Lagermittel des Ventilelementes kann dabei durch in dessen Ober- und/oder Unterseite vorgesehene Ausnehmungen, in welche mindestens ein Federelement eingreifbar ist, verwirklicht sein, oder aber beispielsweise durch Vorsehung von umlaufenden Rücksprüngen oder Verbreiterungen beziehungsweise Rändern oder angesetzten Verbreiterungselementen oder Teilen mit verringertem Durchmesser, so dass durch die dann entstehenden Ringflächen oder Ringflächenabschnitte Lagermittel zur Verfügung gestellt sind, an welchen das mindestens eine Federelement angreifen oder aufhängen kann. Das oder die Federelemente können auch am proximalen und/oder distalen Ende derselben mit dem Deckelteil beziehungsweise Bodenteil, beispielsweise auch durch Klebung, befestigt sein, insbesondere wenn diese als Zugfederelemente ausgebildet sind. Insbesondere kann eine Befestigung an einer Unterseite des Deckelteiles beziehungsweise einer Oberseite des Bodenteils erfolgen, und auf der gegenüberliegenden Seite des oder der Federelemente eine Lagerung vorgesehen sein. Die Befestigung kann beispielsweise erfolgen durch Vorsehung von Einhängungen für mit Endhaken versehene Zugfedern, die zum Beispiel an einem Einhängesteg angeordnet sind, an mindestens einem, bevorzugt beiden Enden des Zugfederelementes.

Das oder die am kolbenartig ausgebildeten Ventilelement angeordnete Lagermittel kann dabei Vorrichtungen umfassen, die der Aufnahme, Haltung oder Positionierung des mindestens einen Federelementes dienen. So können beispielsweise das oder die Lagermittel Vertiefungen oder topfartige Mittel zur Aufnahme von beispielsweise Schraubenfedern, Tellerfedern oder Ringfedern aufweisen, die bevorzugt kreisrund ausgebildet sind, und damit als topfartige Ausnehmungen angesprochen werden können. Auch können die Lagermittel vorstehende Zylinderrohrwandabschnitte aufweisen, gegebenenfalls auch in Kombination mit den vorstehend angesprochenen Ausnehmungen, um eine Haltung beziehungsweise Führung des mindestens einen Federelementes zu verbessern beziehungsweise zu ermöglichen. Auch können dort Befestigungsmittel wie Ösen oder ähnliches vorgesehen sein, beispielsweise zur Befestigung von Schraubenzugfedern. Ein vom Lagermittel umfasster Vorsprung beziehungsweise Ausnehmung ist dabei vorzugsweise derart dimensioniert, dass eine Innenkontur derselben angepasst ist an eine Außenkontur des eingesetzten Federelementes.

Das Gegenlager wird vorzugsweise zur Verfügung gestellt durch das Deckelteil oder das Bodenteil des Gehäuses des erfindungsgemäßen Sprechventiles, je nach dem, ob das Gegenlager am proximalen Ende oder distalen Ende des erfindungsgemäßen Sprechventiles angeordnet ist.

Bei einer Anordnung am proximalen Ende des Sprechventiles können beispielsweise im Bodenteil Ausnehmungen, vorzugsweise kreisrunde und damit als topfartig anzusprechende Ausnehmungen vorgesehen sein zur Aufnahme der Federelemente, beispielsweise von Schraubenfedern et cetera. Entsprechend können dort auch Zylinderrohrwandabschnitte als Vorsprünge auf einer Oberseite des Bodenteiles zugewandt dem kolbenartig ausgebildeten Ventilelement vorgesehen sein, wie dies schon vorstehend in Bezug auf die Lagermittel, zur Verfügung gestellt durch das Ventilelement selbst, beschrieben ist. Auch Kombinationen von Ausnehmungen und Vorsprüngen sind möglich. Das Gegenlager ist dabei vorzugsweise nahe der proximalen Öffnung angeordnet, kann jedoch auch beabstandet von einem Öffnungsrand am Bodenteil angeordnet sein. Stets erfolgt eine Zuordnung des Lagermittels zu dem Gegenlager dahingehend, dass beispielsweise bei Vorsehung mehrerer Federelemente, die gleichmäßig oder ungleichmäßig um einen Umfang des kolbenartigen Federelementes herum angeordnet sind und an diesem angreifen, entsprechende Gegenlager im Bodenteil zur Verfügung gestellt sind.

Entsprechend kann bei der Ausbildung eines Gegenlagers am distalen Ende und damit im Deckelteil des Gehäuses des erfindungsgemäßen Sprechventiles mindestens eine Ausnehmung in einer Oberseite, insbesondere runde, und damit als topfartig anzusprechende vorgesehen sein, in welche das mindestens eine Federelement eingreift und dadurch gehalten, gelagert und/oder positioniert ist. Entsprechend können an einer Unterseite des Deckelteiles, zugewandt dem kolbenartig ausgebildeten Ventilelement, Zylinderrohrwandabschnitte angeordnet sein, auch nur Teilwandabschnitte, wie dies auch bei den übrigen Ausbildungen des Gegenlagers und der Lagermittel der Fall sein kann, in welche Federelemente aufnehmbar sind. Auch bei einer Anordnung des Gegenlagers im Bereich des Deckelteiles ist dieses zugeordnet den Lagermitteln, zur Verfügung gestellt durch das Ventilelement. Dies bedeutet, dass beispielsweise bei Vorsehung mehrerer Federelemente, die am Ventilelement angreifen und dort durch die Lagermittel gelagert sind, entsprechende Gegenlager im Deckelteil vorzusehen sind.

Im Sinne der vorliegenden Erfindung kann jedoch auch vorgesehen sein, dass beispielsweise nur ein einziges Federelement vorgesehen ist. Eine beispielhafte Ausführungsform hierfür wäre bei einer Ausgestaltung des Federelementes als beispielsweise Schraubenfeder oder Spiralfeder oder bei einem ringförmig ausgebildeten geschäumten elastischen Material eine Anordnung derselben an einem Außenumfang, definiert durch die Seitenwandung des kolbenartig ausgebildeten Ventilelementes, so dass dieses zumindest teilweise von dem Federelement umfasst ist. Das oder die Lagermittel, zur Verfügung gestellt durch das kolbenartig ausgebildete Ventilelement, sind dann als beispielsweise in der Seitenwandung oder im Bereich einer auf das proximale Ende des Gehäuses des erfindungsgemäßen Sprechventiles hin angeordnete Verbreiterung oder Rand gebildet, wobei bei einer Anordnung in der Seitenwandung beispielsweise dort auch nur eine Ausnehmung vorgesehen sein kann, in welche das Ende einer beispielsweise Schraubenfeder eingreift. Das Gegenlager kann bei einer derartigen Ausführungsform zur Verfügung gestellt werden durch eine Unterseite des Deckelteiles. Dabei ist es auch möglich, dass das Deckelteil eine insbesondere zentrale Öffnung aufweist, durch welche das Ventilelement bedienbar ist, wobei vorzugsweise das Ventilelement an dessen distalem Ende angeordnet ein Bedienelement aufweist.

Besonders bevorzugt weist das Ventilelement an einer Seitenwandung und/oder einer Unterseite und/oder einer Oberseite angeordnet mindestens ein Lagermittel auf. Besonders bevorzugt ist das mindestens eine Federelement in mindestens einer Ausnehmung des Ventilelementes aufgenommen. Die mindestens eine Ausnehmung ist bevorzugt auf der Unter- und/oder Oberseite des Ventilelementes angeordnet. Das Gegenlager für das mindestens eine Federelement ist vorteilhafterweise zur Verfügung gestellt durch eine Unterseite des Deckelteiles und/oder einer Oberseite eines Bodenteiles.

In einer besonders bevorzugten Ausführungsform umfasst das Ventilelement an seinem der proximalen Öffnung zugewandten unteren Ende eine Verbreiterung, die über eine Seitenwand des Ventilelementes vorspringt und bevorzugt mindestens ein Angreifmittel bildet, das insbesondere zum Halt mindestens eines Federelementes, insbesondere mindestens eines Zugfederelementes, dient.

In einer besonders bevorzugten Ausführungsform der vorliegenden Erfindung ist das Ventilelement zumindest teilweise in dem Federelement angeordnet. Bei einer derartigen Ausführungsform kann, wie bereits vorstehend beschrieben, auch nur ein einziges Federelement vorgesehen sein.

Das Sprechventil weist eine distale Öffnung, in etwa gegenüberliegend einer proximalen Öffnung im Deckelteil auf, insbesondere um eine Bedienung des kolbenartig ausgebildeten Ventilelementes durch die Benutzer zu verbessern beziehungsweise zu erleichtern, liegt erfindungsgemäß das Ventilelement in Offenstellung des Sprechventiles dicht an der Öffnung an. Dies kann beispielsweise dadurch erfolgen, dass ein Dichtungsring am Öffnungsrand des Deckelteiles des Gehäuses vorgesehen ist, oder aber auf der Oberseite oder der Seitenwandung des Ventilelementes vorgesehen ist. Auch können sowohl am Ventilelement als auch am Deckelteil entsprechende Dichtungselemente angeordnet sein, die insbesondere auch zusammenwirken können, beispielsweise durch Einpassung ineinander. Es kann auch vorgesehen sein, dass beispielsweise durch 2-Komponenten-Spritzpressen im Bereich der Oberseite oder der Seitenwandung das Ventilelement ein weicheres, elastisches Kunststoffmaterial aufweist, über welches dann eine Dichtung zur Verfügung gestellt ist, gegebenenfalls in Kombination mit entsprechend im Bereich der distalen Öffnung im Deckelteil angeordneten weiteren Dichtmitteln, oder umgekehrt.

Entsprechend kann eine Abdichtung des proximalen Endes bei Überführung des Ventilelementes aus einer Offenstellung in eine Schließstellung erfolgen. Auch hier können Dichtmittel insbesondere am Öffnungsrand oder im Bereich der proximalen Öffnung angesehen sein, welche mit dem Ventilelement zusammenwirken. Auch können am Ventilelement, das heißt insbesondere an dessen proximalen Ende, entsprechende Dichtmittel vorgesehen sein. Auch können sowohl am Dichtelement, insbesondere an dessen proximalen Ende oder aber im Bereich der Seitenwandungen, Dichtelemente vorgesehen sein, ebenso wie im Bereich der proximalen Öffnung oder unmittelbar an dieser, wobei dann die jeweiligen Dichtelemente auch zusammenwirken können, beispielsweise durch Bildung eines Formschlusses. Auch kann das Ventilelement an seinem proximalen Ende, beispielsweise an der der proximalen Öffnung zugewandten Unterseite, oder aber auch im Bereich der Seitenwandungen, beispielsweise erhältlich durch ein 2-Komponenten-Spritzpressen, ein elastisches Material aufweisen, durch welches eine Dichtung erzielbar ist. Hier gilt das vorstehend in Bezug auf die Abdichtung der Öffnung im Deckelteil, die der proximalen Öffnung vorzugsweise gegenüberliegt, Ausgeführte entsprechend.

Vorzugsweise weist das Deckelteil des Gehäuses des erfindungsgemäßen Sprechventiles mindestens ein Rückhaltemittel für das Ventilelement auf. Das Rückhaltmittel kann dabei insbesondere einstückig mit dem Deckelteil ausgebildet sein, und beispielsweise zur Verfügung gestellt sein durch die Oberseite des Deckelteiles selbst. Eine entsprechende Ausführungsform weist beispielsweise eine vorzugsweise zentrale Öffnung in der Oberseite des Deckelteiles auf, durch welche das Ventilelement etwas herausragt, gegebenenfalls auch nur ein auf dem distalen Ende desselben angeordnetes Bedienelement zur Erleichterung der Bedienung des Sprechventiles durch einen Benutzer. Dann dient die Unterseite des Randbereiches des Deckelteiles um diese Öffnung herum als Rückhaltmittel. Es können jedoch auch andere Arten von Rückhaltmitteln vorgesehen sein, welche beispielsweise an der Unterseite des Deckelteiles, zugewandt der proximalen Öffnung des Sprechventiles, angeordnet sind, und den Federweg des Ventilelementes bei Überführung aus der Schließstellung in die Offenstellung beschränken. Letzteres ist auch die Grundfunktion, die das oder die Rückhaltemittel im Sinne der vorliegenden Erfindung erfüllen müssen. Beispielsweise kann als Rückhaltemittel an der Unterseite des Deckelteiles angeordnet ein kreisrunder Dichtring dienen, der mit einer Oberseite oder Seitenwandung des Ventilelementes zusammenwirkt und hierdurch den Federweg begrenzt.

Besonders bevorzugt weist das Gehäuse mindestens eine zu einer zentralen Hauptachse senkrecht und/oder parallel zu dieser ausgerichtete Öffnung auf. Vorzugsweise sind mehrere Öffnungen vorgesehen, welche weiter bevorzugt gleichmäßig am Außenumfang des Gehäuses, vorzugsweise am Außenumfang des Deckelteiles, angeordnet sind. Alternativ oder zusätzlich hierzu können auch Öffnungen im Deckelteil vorgesehen sein, welche parallel zu der zentralen Hauptachse ausgerichtet sind. Dies lässt sich verwirklichen, wenn das Deckelteil das Bodenteil überspringt, so dass im Bereich des Übersprungs Öffnungen in einer Ebene parallel zur Fläche der Oberseite des Deckelteiles und versetzt auf das proximale Ende des erfindungsgemäßen Sprechventiles hin möglich sind vorzusehen. Diese weist mit ihrer Mündung dann auf die Haut des Trägers hin. Es kann auch vorgesehen sein, dass nur eine Öffnung vorgesehen ist, wenn diese beispielsweise siebartig ausgebildet ist. Im Sinne der vorliegenden Erfindung ist dabei dann das Sieb selbst als eine einzige Öffnung angesprochen, die einzelnen Öffnungen des Siebes selbst werden dabei nicht berücksichtigt.

Die mindestens eine senkrecht und/oder parallel zu der zentralen Hauptachse ausgerichtete Öffnung kann jedwede Form aufweisen, und beispielsweise kreisrund, dreieckig oder viereckig, insbesondere quadratisch oder rechteckig, aber auch ringförmig ausgestaltet sein. Aber auch jede andere Öffnungsform ist möglich. Beispielsweise kann eine Seitenwandung des Deckelteiles, die parallel zu zentralen Hauptachse ausgerichtet ist, eine Vielzahl von rechteckigen Öffnungen aufweisen, die nur durch Stege zwischen der Oberseite des Deckelteiles und einer Unterseite desselben beziehungsweise einer Oberseite des Bodenteils angeordnet sind, und so eine Verbindung zwischen Deckelteil und Bodenteil des Gehäuses zur Verfügung stellen. Vorteilhafterweise sind mehrere durch Stege und/oder Wandungsabschnitte voneinander getrennte Öffnungen vorgesehen. Wandungsabschnitte sind dabei beispielsweise die Bereiche, die zwischen zwei benachbarten kreisrund ausgebildeten Öffnungen in der Seitenwandung des Deckelteiles vorliegen, oder aber im Bereich der Wandung eines Übersprungs des Deckelteiles, wenn Öffnungen in einer Ebene parallel zur Oberseite des Deckelteiles und versetzt auf das proximale Ende zu vorgesehen werden.

Eine Verbindung zwischen Deckelteil und Bodenteil kann auch zur Verfügung gestellt sein durch Stege oder eine Begrenzungswand mit Öffnungen, die am Deckelteil und Bodenteil angreifen und, insbesondere nahe der Außenwandung des Filters, im Inneren des Gehäuses angeordnet ist/sind. Eine Begrenzungswand oder Stege können aber auch die Funktion ausüben, eine Aufnahme für den Filter und/oder eine Führung für das Ventilelement zur Verfügung zu stellen, wobei dann keine Verbindung von Deckelteil und Bodenteil erfolgen muss. Beispielsweise können die Stege beziehungsweise die Begrenzungswand an der Unterseite des Deckelteils angeordnet sein, und sich auf das Bodenteil hinzu erstrecken, aber vor der Oberseite desselben enden, und umgekehrt.

Durch die vorliegende Erfindung ist ein Sprechventil zur Verfügung gestellt, welches eine sichere Schließung ermöglicht, ohne dass die Feuchtetauscherfunktion des Filters aufgrund Kompression und Dekompression desselben behindert wird. Dabei ist das Ventilelement für den Benutzer sicher führbar und vermittelt durch den Kontakt des Ventilelementes bei Überführung in die Schließstellung mit dem Ventilsitz an diesen auch eine Rückmeldung, dass nun in Schließstellung ein Sprechen ermöglicht ist.

Die Erfindung wird anhand der nachfolgenden Figuren näher erläutert. Es zeigen:
- Fig. 1:: eine erste Ausführungsform des erfindungsgemäßen Sprechventiles;
- Fig. 2:: eine zweite Ausführungsform des erfindungsgemäßen Sprechventiles; und
- Fig. 3:: eine dritte Ausführungsform des erfindungsgemäßen Sprechventiles.

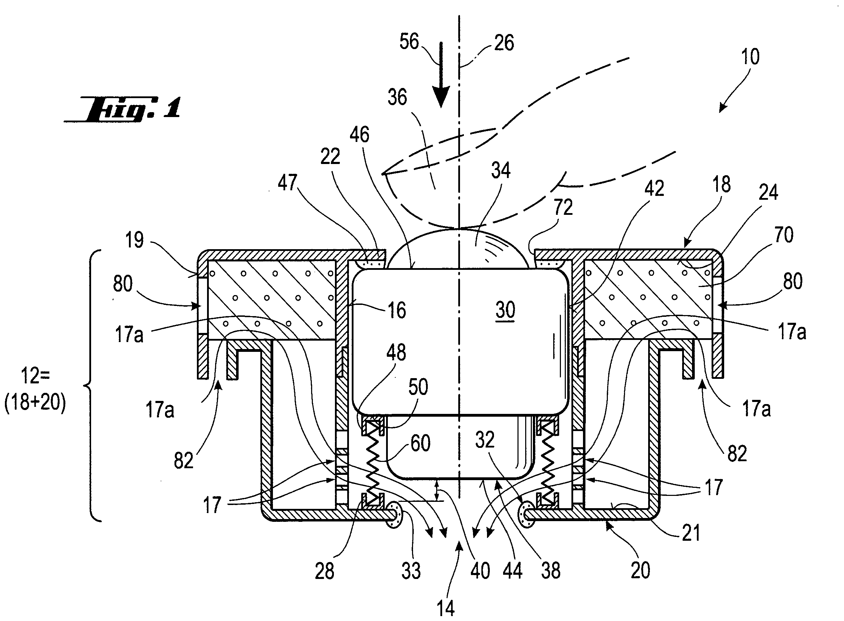

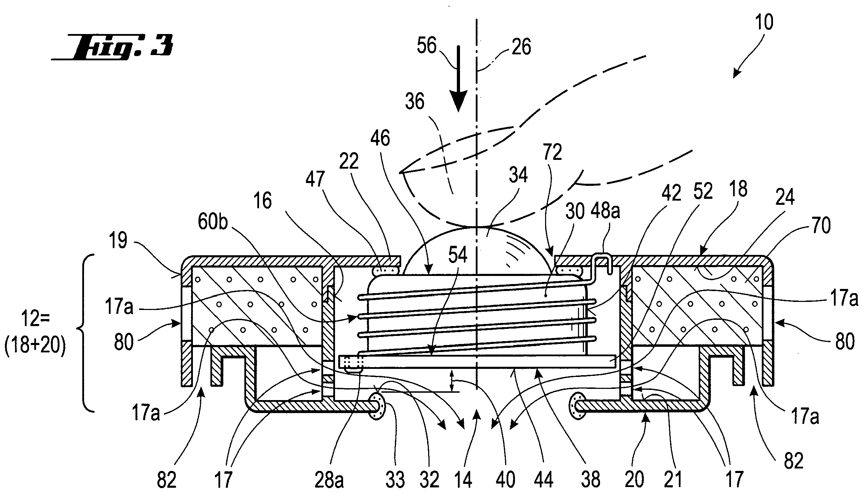

Die Fig. 1 und Fig. 3 zeigen die drei Ausführungsformen des erfindungsgemäßen Sprechventiles jeweils in einer Schnittansicht. Zunächst sei vorausgeschickt, dass die Erfindung nicht auf die in den Figuren gezeigten Merkmalskombinationen beschränkt ist. Vielmehr sind die jeweils in der Beschreibung einschließlich der Figurenbeschreibung offenbarten Merkmale mit denjenigen in den Figuren angegebenen Merkmalen kombinierbar. Insbesondere ist die Ausbildung des in den Figuren gezeigten Ventilsitzes mit einer Dichtung nur eine der möglichen Ausgestaltungsformen. Auf eine Dichtung am Ventilsitz kann auch verzichtet werden. Darüber hinaus kann jede Art von Federelement, insbesondere auch mehrere Federelemente, vorgesehen sein und auf jede mögliche Art und Weise angeordnet sein, um eine Bewegbarkeit eines kolbenartig ausgebildeten Ventilelementes zu ermöglichen. Das mindestens eine Federelement kann zum Beispiel auch aus einem elastischen Kunststoffmaterial, insbesondere geschäumt, ausgebildet sein. Auch kann das Filtermaterial nicht nur unmittelbar benachbart, auch anliegend an einer Unterseite eines Deckelteiles des Gehäuses der erfindungsgemäßen Sprechventile angeordnet sein, sondern sich auch bis zu einer Oberseite eines Bodenteiles des Gehäuses, auch anliegend, erstrecken, oder aber auch jede andere Ausdehnung zwischen diesen beiden Grenzen einnehmen. Schließlich ist auch darauf hinzuweisen, dass die in den Ansprüchen aufgenommenen Bezugszeichen in keiner Weise den Schutzbereich der vorliegenden Erfindung beschränken sollen, sondern lediglich auf die in den Figuren gezeigten Ausführungsformen verweisen. In den vorgeschlagenen drei alternativen Ausführungsformen sind im Übrigen zur Vereinfachung gleiche Bezugszeichen für gleiche Merkmale verwendet.

Fig. 1 zeigt nun in vergrößerter Schnitteinsicht die erste Ausführungsform des erfindungsgemäßen Sprechventiles 10 mit einem Gehäuse 12 in Offenstellung. Das Gehäuse 12 weist eine körpernahe proximale Öffnung 14 auf. Das Sprechventil 10 wird beispielsweise auf eine hier nicht gezeigte Trachealkanüle oder aber ein Pflaster, welches über das Stoma des Trägers geklebt ist, aufgesteckt. Hierfür sind üblich Konnektoren, auch nach DIN-Normen, beispielsweise mit 15 oder 22 mm Durchmesser, bekannt.

Das Gehäuse 12 weist in Bezug zu einer zentralen Hauptachse 26 senkrecht ausgerichtete Öffnungen 80 und parallel zu dieser ausgerichtete Öffnungen 82 auf, durch welche Luft 17a durch einen Filter 70 in das Innere des Gehäuse 12 und über die proximale erste Öffnung 14 in das Stoma eines Benutzers einströmen kann in Offenstellung des Sprechventiles 10, als auch in umgekehrter Richtung über die proximale erste Öffnung 14 durch die Öffnungen 80 und 82 nach Durchtritt durch den Filter 70 ausströmen kann. Die Öffnungen 80 und 82 (letztere können zum Beispiel auch ausgelassen werden) sind zum Beispiel siebartig oder kreisrund ausgebildet und gleichmäßig über den Außenumfang des Deckelteils 18 in der seitlichen Wandung 19 und/oder in einer Fläche in der Ebene der Öffnungen 82 verteilt angeordnet. Durch Stege oder sonstige Wandabschnitte zwischen den Öffnungen 80 und/oder 82 ist eine sichere Verbindung des Deckelteils 18 mit dem Bodenteil 20 zur Verfügung gestellt. Die Luft strömt dabei durch einen unterhalb eines Endes einer Begrenzungswand 16 angeordneten Spalt 40, welcher ausgebildet ist zwischen einem unteren Ende 38 eines Ventilelementes 30 mit einer Seitenwandung 42, welches kolbenartig ausgebildet im Gehäuse 12 angeordnet ist, und einem Ventilsitz 32, welcher eine Dichtung 33 aufweist, wobei der Spalt 40 ein Ringspalt ist. Zwischen einem unteren Ende 38 des Ventilelementes 30 und dem Ventilsitz 32 ist in Schließstellung bei einer Bewegung des Ventilelementes 30 über ein Bedienmittel 34 mittels eines virtuell angedeuteten Fingers 36 eines Benutzers in Richtung des Pfeiles 56 bis zur Anlage des unteren Endes 38 auf dem Ventilsitz 32 ein Luftstrom in das hier nicht gezeigte Stoma des Patienten durch die proximale erste Öffnung 14 verhindert und hierdurch ein Sprechen für den Benutzer ermöglicht, insbesondere soweit dieser bei einer Entfernung des Larynx eine Stimmprothese eingesetzt hat. Die Begrenzungswand 16 dient zum Beispiel der Zurverfügungstellung einer Aufnahme für den Filter 70, andererseits der Luftführung auf den Spalt 40 zu. Sie ist vorzugsweise als umlaufender Zylinderrohrwandabschnitt an einer Unterseite 24 des Deckelteils 18 in Richtung auf die proximale Öffnung 14 hin ausgebildet. Dabei kann die Begrenzungswand 16 Öffnungen 17 aufweisen, durch die Luftströme 17a in Richtung auf den Spalt 40 zuströmen können, oder umgekehrt ausgeatmet werden können. Dann kann durch zwischen beispielsweise kreisrunden oder siebartigen Öffnungen 17 in der Begrenzungswand 16 angeordnete Stege oder Wandungsabschnitte eine (weitere) Verbindung zwischen Deckelteil 18 und Bodenteil 20 zur Stabilisierung des Gehäuses 12 erfolgen. Es können aber auch keine Öffnungen vorgesehen sein, und dabei insbesondere etwa in Höhe eines Rücksprunges 50 die Begrenzungswand 16 enden zur Ermöglichung eines Luftdurchtrittes in Richtung auf den Spalt 40 zu.

Das kolbenartig mit einem zylindrischen Mittelteil ausgebildete Ventilelement 30 weist zum unteren Ende 38 hin den umlaufenden Rücksprung 50 auf, wodurch letztendlich ein verringerter Durchmesser des Endes 38 des Ventilelementes 30 zur Schließung des Ventilsitzes 32 zur Verfügung gestellt ist, so dass auch die proximale erste Öffnung 14 kleiner ausgebildet sein kann.

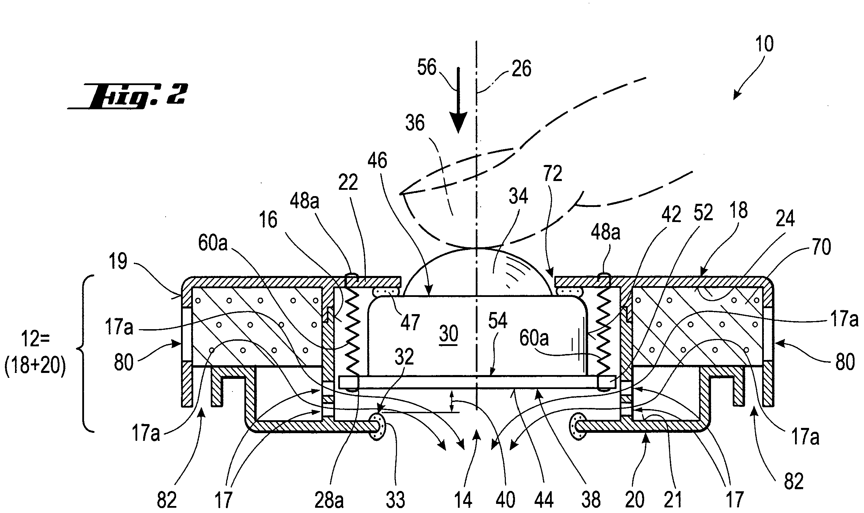

Die durch den Rücksprung 50 des Ventilelementes 30 gebildete ringförmige Fläche bildet ein Lagermittel 48 für Federelemente 60, hier Druckfederelemente 60. Auch wenn die ringförmige Fläche als Lagermittel 48 ausreichend ist, können dort zum Beispiel wie gezeigt, topfartige Aufnahmen das Lagermittel 48 ergänzen. Das oder die Lagermittel 48 könnten beispielsweise auch als Ausnehmungen im Bereich des Rücksprungs 50 im Ventilelement 30 ausgebildet sein. Dabei weist die in Fig. 1 gezeigt erste Ausführungsform des Sprechventiles 10 mehrere Federelemente 60 auf, welche um das durch den Rücksprung 50 im Durchmesser verringerte untere Ende 38 des Ventilelementes 30 herum gleichmäßig verteilt angeordnet sind, und auf der durch den Rücksprung 50 ausgebildeten ringförmigen Fläche gelagert sind. Es kann jedoch alternativ auch nur ein einziges Federelement 60 vorgesehen sein, bei mehreren können beispielsweise vier, sechs oder acht, bevorzugt regelmäßig beabstandet voneinander, vorgesehen sein. Als zum Beispiel einziges Federelement könnte wie auch bei den weiteren Ausführungsformen gemäß den Fig. 2 und 3 ein solches aus einem hinreichend elastischen, geschäumten Kunststoffmaterial eingesetzt werden, welches als Feinflter und ringförmig ausgebildet ist.

Als Gegenlager 28 für die Federelemente 60 fungiert eine Oberseite 21 eines Bodenteiles 20, wobei Halte- beziehungsweise Positioniermittel auf der Oberseite 21 zur Halterung und/oder Positionierung der Federelemente 60 vorgesehen sein können, beziehungsweise in Form von auf der Oberseite 21 des Bodenteiles 20 angeordneten Zylinderrohrwandabschnitten, die in ihrer Abmessung einer Außenkontur der Federelemente 60 angepasst sind. Das untere Ende 38 des verjüngten unteren Teiles des Ventilelementes 30 ist eben ausgebildet mit einer Unterseite 44, welche über Rundungen in die Seitenwandung des Ventilelementes 30 übergeht. Im Bereich dieser Rundungen erfolgt über eine ringförmige Dichtung 33 des Ventilsitzes 32 der Kontakt des Ventilelementes 30 mit dem Ventilsitz 32 zur Unterbindung eines Luftstromes durch den Spalt 40 des erfindungsgemäßen Sprechventiles 10.

In der in Fig. 1 gezeigten Offenstellung wird aufgrund der Federkraft der Federelementes 60 der kolbenartige Ventilkörper 30 in Richtung auf die Unterseite 24 des Deckelteiles 18 des Gehäuses 12 zu gedrückt. Durch am Deckelteil 18 vorgesehene Rückhaltemittel 22, welche vorzugsweise umlaufend um das Bedienelement 34 des Ventilelementes 30 angeordnet sind, aber auch beispielsweise stegartig oder sonst wie vorsprungartig ausgebildet sein können, wird das Ventilelement 30 im Gehäuse 12 in Position gehalten. Bevorzugt und wie in Fig. 1 gezeigt ist ein Rückhaltemittel 22 vorgesehen, welches in das Deckelteil 18 einstückig integriert ist, so dass die Oberseite des Deckelteiles 18 in Aufsicht als ebene Fläche mit einer vorzugsweise kreisrunden Öffnung 72 erscheint. Das Deckelteil 18 umfasst die seitliche Wandung 19, die in etwa parallel zu der Hauptachse 26 ausgebildet ist. Vorzugsweise ist dabei auf einer Oberseite 46 des Ventilelementes 30, insbesondere bei Vorsehung des Bedienelementes 34 umlaufend um dieses, ein Dichtmittel 47 angeordnet, um eine Dichtung zwischen Ventilkörper 30 und dem Deckelteil 18 mit den Rückhaltemitteln 22 zur Verfügung zu stellen. Alternativ können auch auf der Unterseite 24 des Deckelteiles 18 im Bereich der Rückhaltemittel 22 Dichtungsmaterialien angeordnet sein. In einer weiter bevorzugten Ausführungsform können Dichtungsmaterialien sowohl auf der Unterseite 24 des Deckelteiles 18 im Bereich der Rückhaltemittel 22 als auch auf der Oberseite 46 des Ventilelementes 30 angeordnet sein. Das Dichtungsmaterial kann dabei einstückig mit dem Deckelteil 18 des Gehäuses 12 und/oder aber mit dem Ventilelement 30 verbunden sein. Dies kann beispielsweise durch ein 2-Komponenten-Spritzpressen in der Fertigung des Sprechventiles 10 erfolgen.

Der Filter 70 wird bei dem Sprechventil 10 weder komprimiert noch sonst wie bewegt. Der Filter 70 umfasst das Ventilelement. Der Filter 70 ist der Öffnung 72 zugeordnet und damit unmittelbar benachbart, auch gegebenenfalls anliegend, gegebenenfalls auch befestigt, der Unterseite 24 des Deckelteiles 18 im Gehäuse 12 aufgenommen. Der Filter 70 ist beabstandet von der proximalen ersten Öffnung 14 und damit der Oberseite 21 des Bodens 20 im Gehäuse 12 angeordnet.

Das Lagermittel 48 und das Gegenlager 28, jeweils auch mehrere, können in jeder erdenklichen Art und Weise ausgebildet sein. Diese können beispielsweise zapfenförmige Vorsprünge mit einer Ausnehmung zur Aufnahme der Federelemente 60 umfassen, oder aber umlaufende Wände oder aber eine umlaufende Ringfläche oder zum Beispiel topfartige Ausnehmungen im Ventilelement 30, ausgehend von einer Oberfläche des Rücksprungs 50 in den Körper des Ventilelementes 30 hineinreichend, im Falle des Lagermittels 48 sein, wobei im erstgenannten Falle eine gegenüberliegende Wand durch die Seitenwandung 42 im unteren, im Durchmesser verringerten Ende 38 des Ventilelementes 30 zur Verfügung gestellt ist. Über das beziehungsweise die Lagermittel 48 und Gegenlager 28 kann auch eine Verbindung zwischen Bodenteil 20 und Deckelteil 18 zur Verfügung gestellt sein.

Nach Betätigung in Richtung des Pfeiles 56 durch den Benutzer wird der Spalt 40 zwischen unterem Ende 38 beziehungsweise der Unterseite 44 des Ventilelementes 30 und dem Ventilsitz 32 mit der dort angeordneten Dichtung 33 geschlossen, und sobald der Benutzer nicht mehr wünscht zu sprechen, wird durch Entlastung, das heißt Entfernung des Fingers 36 vom Bedienmittel 34 oder Verringerung des durch den Finger 36 ausgeübten Druckes auf die Federelemente 60 das Ventilelement 30 durch die Federelemente 60 wieder in die Offenstellung gemäß Fig. 1 zurückbewegt.

Fig. 2 zeigt eine alternative zweite Ausführungsform des erfindungsgemäßen Sprechventiles 10, welche im Unterschied zu derjenigen gemäß Fig. 1 bedeutend niedriger bauend ausgelegt ist. Die Anordnung des Filters 70 entspricht derjenigen in der ersten Ausführungsform. Im Wesentlichen ist das Ventilelement 30 anders ausgebildet, indem es zusätzlich an seinem unteren Ende 38 eine Verbreiterung 52 aufweist, welche über die Seitenwandung 42 des Ventilelementes 30 übersteht und ein oder mehrere Angreifmittel 54 bildet, welches beziehungsweise welche vorzugsweise umlaufend um die Seitenwandung 42 des Ventilelementes 30 ausgebildet ist beziehungsweise sind. Die Federelemente, hier Zugfederelemente 60, bevorzugt mindestens zwei, weiter bevorzugt mindestens drei, sind an der Unterseite 24 des Deckelteils 18 an Befestigungsstellen 48a fest befestigt. Die Verbreiterung 52 kann dabei eine geschlossene ringförmige Fläche als Angreifmittel 54 zur Verfügung stellen, sie kann aber auch zum Beispiel Rücksprünge aufweisen, so dass durch die zwischen Rücksprüngen angeordneten Flächen die Angreifmittel 54 zur Verfügung gestellt sind, soweit eine Abdichtung des Ventilsitzes 32 in Schließstellung des erfindungsgemäßen Sprechventiles 10 sichergestellt ist. Auch können zur Aufnahme von Federelementen 60 Lagermittel zum Beispiel in Form von kreisrunden Ausnehmungen oder vorspringenden Zylinderrohrwandabschnitten auf eine dem Deckelteil 18 zugewandten Oberseite der Verbreiterung 52 vorgesehen seien. Das oder die Angreifmittel 54 stellen Befestigungsstellen 28a für mehrere, vorzugsweise vier, sechs oder acht, bevorzugt gleichmäßig um den Außenumfang des Ventilelementes 30 angeordnete Zugfederelemente 60a, zum Beispiel als Schraubenzugfedern ausgestaltet, mit geringem Durchmesser dar, wobei Befestigungsstellen durch die Unterseite 24 resp. Rückhaltemittel 22 des Deckelteiles 18 gebildet sind. Die Funktionsweise in Offen- und Schließstellung der in Fig. 2 gezeigten zweiten Ausführungsform entspricht derjenigen in Fig. 1 gezeigten.

Fig. 3 zeigt eine alternative dritte Ausführungsform des erfindungsgemäßen Sprechventiles 10. Im Unterschied zu der in Fig. 2 gezeigte Ausführungsform ist eine einzige Schraubenfeder 60b vorgesehen, in welcher das Ventilelement 30 teilweise aufgenommen ist, wobei als eine Befestigungsstelle 28b für die Schraubenfeder 60b die Unterseite 24 des Deckelteiles 18 dient. Die andere Befestigungsstelle 28b für die Schraubenfeder 60b wird durch die Verbreiterung 52 des Ventilelementes 30, entsprechend wie in Fig. 2 gezeigt, gebildet.

## Patentansprüche

1. Sprechventil (10) für Laryngektomierte oder Tracheotomierte mit einem Gehäuse (12) mit einer proximalen Öffnung (14) und mindestens einem im Gehäuse (12) zumindest teilweise aufgenommenen Filter (70) und mit einem kolbenartig ausgebildeten Ventilelement (30), wobei der Filter (70) das Ventilelement (30) zumindest teilweise umgibt, wobei das Ventilelement (30) auf einen an der proximalen Öffnung (14) angeordneten Ventilsitz (32) zur Erzeugung einer Schließstellung bewegbar ist und federnd in eine Offenstellung des Sprechventiles (10) rückführbar ist, wobei das Gehäuse (12) ein Deckelteil (18) umfasst, welches eine Öffnung (72) für das Ventilelement (30) aufweist, wobei das Ventilelement (30) in Offenstellung des Sprechventiles dicht an der Öffnung (72) anliegt, und wobei das Ventilelement (30) ein unteres, der proximalen Öffnung (14) zugewandtes Ende (38) aufweist, welches in Offenstellung dem Ventilsitz (32) zugeordnet ist und mit dem Ventilsitz (32) einen Spalt (40) bildet, durch den ein Luftstrom führbar ist.

2. Sprechventil gemäß Anspruch 1, **dadurch gekennzeichnet, dass** das Ventilelement (30) über mindestens ein Federelement (60) im Gehäuse (12) gelagert ist.

3. Sprechventil gemäß einem oder mehreren der vorhergehenden Ansprüche, **dadurch gekennzeichnet, dass** das Deckelteil (18) mindestens ein Rückhaltmittel (22) für das Ventilelement (30) aufweist.

4. Sprechventil gemäß Anspruch 2 oder gemäß Anspruch 3, wenn abhängig vom Anspruch 2, **dadurch gekennzeichnet, dass** das Ventilelement (30) an einer Seitenwandung (42) und/oder einer Unterseite (44) und/oder einer Oberseite (46) angeordnet mindestens ein Lagermittel (48) für das mindestens eine Federelement (60) aufweist.

5. Sprechventil gemäß Anspruch 2 oder gemäß einem der Ansprüche 3 oder 4, wenn abhängig vom Anspruch 2, **dadurch gekennzeichnet, dass** das mindestens eine Federelement (60) in mindestens einer Ausnehmung des Ventilelementes (30) aufgenommen ist.

6. Sprechventil gemäß Anspruch 4 oder gemäß Anspruch 5, wenn abhängig vom Anspruch 4, **dadurch gekennzeichnet, dass** ein Gegenlager (28) für das mindestens eine Federelement (60) durch eine Unterseite (24) des Deckelteiles (18) und/oder eine Oberseite (21) eines Bodenteiles (20), welches vom Gehäuse (12) umfasst ist, zur Verfügung gestellt ist.

7. Sprechventil gemäß einem oder mehreren der vorhergehenden Ansprüche, **dadurch gekennzeichnet, dass** das Gehäuse (12) mindestens eine zu einer zentralen Hauptachse (26) senkrecht und/oder parallel zu dieser ausgerichtete Öffnung (80, 82) aufweist.

8. Sprechventil gemäß Anspruch 7, **dadurch gekennzeichnet, dass** mehrere durch Stege und/oder Wandungsabschnitte voneinander getrennte Öffnungen (80, 82) vorgesehen sind.

9. Sprechventil gemäß einem oder mehreren der vorhergehenden Ansprüche, **dadurch gekennzeichnet, dass** das Ventilelement (30) an seinem der proximalen Öffnung (14) zugewandten unteren Ende (38) eine Verbreiterung (52) umfasst, die über eine Seitenwandung (42) des Ventilelementes (30) vorspringt.

10. Sprechventil gemäß Anspruch 9, wenn abhängig vom Anspruch 2, **dadurch gekennzeichnet, dass** das Ventilelement (30) zumindest teilweise in dem Federelement (60) angeordnet ist.

## Claims

1. Speech valve (10) for laryngectomized or tracheotomized persons, comprising a housing (12) with a proximal opening (14) and a least one filter (70) at least partially received in the housing (12), and a piston-like valve element (30), wherein the filter (70) at least partially encompasses the valve element (30), wherein the valve element (30) can be moved onto a valve seat (32) disposed on the proximal opening (14) in order to generate a closure position, and can be returned to an open position of the speech valve (10) in a spring-loaded manner, wherein the housing (12) comprises a cover part (18) having an opening (72) for the valve element (30), wherein the valve element (30) in an open position closely abuts the opening (72), and wherein the valve element (30) has a lower end (38) facing the proximal opening (14), which is in an open position allocated to the valve seat (32) and forms a gap (40) with the valve seat (32), through which an airflow can be conducted.

2. Speech valve according to claim 1, **characterized in that** the valve element (30) is supported in the housing (12) via at least one spring element (60).

3. Speech valve according to one or more of the preceding claims, **characterized in that** the cover part (18) has at least one retaining means (22) for the valve element (30).

4. Speech valve according to claim 2 or according to claim 3, if dependent on claim 2, **characterized in that** the valve element (30) has at least one bearing means (48) for the at least one spring element (60) disposed on a side wall (42) and/or an undersurface (44) and/or an upper surface (46) thereof.

5. Speech valve according to claim 2, or according to one of the claims 3 or 4, if dependent of claim 2, **characterized in that** the at least one spring element (60) is received in at least one recess of the valve element (30).

6. Speech valve according to claim 4 or according to claim 5, if dependent of claim 4, **characterized in that** a counter-bearing (28) for the at least one spring element (60) is provided by an undersurface (24) of the cover part (18) and/or an upper surface (21) of a bottom part (20), which is comprised by the housing (12).

7. Speech valve according to one ore more of the preceding claims, **characterized in that** the housing (12) has at least one opening (80, 82) oriented in a perpendicular and/or parallel manner towards a central main axis (26).

8. Speech valve according to claim 7, **characterized in that** a number of openings (80, 82) are provided, separated from one another by bridges and/or wall sections.

9. Speech valve according to one or more of the preceding claims, **characterized in that** the valve element (30) has a widening (52) on its lower end (38) facing the proximal opening (14), which extends beyond a side wall (42) of the valve element (30).

10. Speech valve according to claim 9, if dependent of claim 2, **characterized in that** the valve element (30) is disposed at least partially in the spring element (60).

## Revendications

1. Valve phonique (10) pour des patients laryngectomisés ou trachéotomisés avec un boîtier (12) présentant une ouverture proximale (14) et au moins un filtre (70) logé au moins partiellement dans le boîtier (12) ainsi qu'un élément de soupape (30) réalisé à la manière d'un piston, dans laquelle le filtre (70) entoure au moins partiellement l'élément de soupape (30), dans laquelle l'élément de soupape (30) peut être amené sur un siège de soupape (32) disposé à l'ouverture proximale (14) pour produire une position fermée et peut être ramené élastiquement dans une position ouverte de la valve phonique (10), dans laquelle le boîtier (12) comprend une partie de couvercle (18), qui présente une ouverture (72) pour l'élément de soupape (30), dans laquelle l'élément de soupape (30) s'applique de façon hermétique sur l'ouverture (72) dans la position ouverte de la valve phonique, et dans laquelle l'élément de soupape (30) présente une extrémité inférieure (38) tournée vers l'ouverture proximale (14), qui en position ouverte est associée au siège de soupape (32) et qui forme avec le siège de soupape (32) une fente (40), à travers laquelle un courant d'air peut être conduit.

2. Valve phonique selon la revendication 1, **caractérisée en ce que** l'élément de soupape (30) est monté par au moins un élément de ressort (60) dans le boîtier (12).

3. Valve phonique selon une ou plusieurs des revendications précédentes, **caractérisée en ce que** la partie de couvercle (18) présente au moins un moyen de retenue (22) pour l'élément de soupape (30).

4. Valve phonique selon la revendication 2 ou selon la revendication 3, lorsqu'elle dépend de la revendication 2, **caractérisée en ce que** l'élément de soupape (30) présente au moins un moyen d'appui (48) pour ledit au moins un élément de ressort (60), disposé sur une paroi latérale (42) et/ou sur un côté inférieur (44) et/ou sur un côté supérieur (46).

5. Valve phonique selon la revendication 2 ou selon une des revendications 3 ou 4, lorsqu'elle dépend de la revendication 2, **caractérisée en ce que** ledit au moins un élément de ressort (60) est logé dans au moins un évidement de l'élément de soupape (30).

6. Valve phonique selon la revendication 4 ou selon la revendication 5, lorsqu'elle dépend de la revendication 4, **caractérisée en ce qu'**un contre-appui (28) pour ledit au moins un élément de ressort (60) est formé par un côté inférieur (24) de la partie de couvercle (18) et/ou par un côté supérieur (21) d'une partie de fond (20), qui est comprise dans le boîtier (12).

7. Valve phonique selon une ou plusieurs des revendications précédentes, **caractérisée en ce que** le boîtier (12) présente au moins une ouverture (80, 82) orientée perpendiculairement à un axe principal central (26) et/ou parallèlement à celui-ci.

8. Valve phonique selon la revendication 7, **caractérisée en ce qu'**il est prévu plusieurs ouvertures (80, 82) séparées l'une de l'autre par des nervures et/ou des parties de paroi.

9. Valve phonique selon une ou plusieurs des revendications précédentes, **caractérisée en ce que** l'élément de soupape (30) comporte, à son extrémité inférieure (38) tournée vers l'ouverture proximale (14), une région élargie (52), qui est saillante au-delà d'une paroi latérale (42) de l'élément de soupape (30).

10. Valve phonique selon la revendication 9, lorsqu'elle dépend de la revendication 2, **caractérisée en ce que** l'élément de soupape (30) est disposé au moins en partie dans l'élément de ressort (60).
